(19) Europäisches Patentamt
European Patent Office
Office européen des brevets

(11) **EP 3 463 051 B1**

(12) **FASCICULE DE BREVET EUROPEEN**

(45) Date de publication et mention
de la délivrance du brevet:
**04.12.2024 Bulletin 2024/49**

(21) Numéro de dépôt: **17731238.6**

(22) Date de dépôt: **31.05.2017**

(51) Classification Internationale des Brevets (IPC):
***A61B 5/00*** *(2006.01)*

(52) Classification Coopérative des Brevets (CPC):
**A61B 5/6803; A61B 5/163;** A61B 5/02055;
A61B 5/1103; A61B 5/1114; A61B 5/14532;
A61B 5/18; A61B 5/7445; A61B 5/7455;
A61B 2503/22; A61B 2560/0242; A61B 2562/0219

(86) Numéro de dépôt international:
**PCT/FR2017/051362**

(87) Numéro de publication internationale:
**WO 2017/207925 (07.12.2017 Gazette 2017/49)**

(54) **DISPOSITIF CONNECTÉ DE SUIVI COMPORTEMENTAL D'UN INDIVIDU ET PERMETTANT LA DÉTECTION ET/OU LA PRÉVENTION D'UNE ANOMALIE**

VERBUNDENE VORRICHTUNG ZUR VERHALTENSÜBERWACHUNG EINES INDIVIDUUMS UND ZUR DETEKTION UND/ODER VERHINDERUNG EINER ANOMALIE

CONNECTED DEVICE FOR BEHAVIOURAL MONITORING OF AN INDIVIDUAL AND FOR DETECTING AND/OR PREVENTING AN ANOMALY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.05.2016 FR 1654922**

(43) Date de publication de la demande:
**10.04.2019 Bulletin 2019/15**

(73) Titulaire: **Euro Protection Surveillance**
**67100 Strasbourg (FR)**

(72) Inventeur: **PEYRARD, Philippe**
**06600 Antibes (FR)**

(74) Mandataire: **Cabinet Nuss**
**10, rue Jacques Kablé**
**67080 Strasbourg Cedex (FR)**

(56) Documents cités:
**EP-A1- 2 061 026      US-A1- 2001 028 309**
**US-A1- 2009 105 605      US-A1- 2010 134 761**

EP 3 463 051 B1

**Description**

DOMAINE TECHNIQUE DE L'INVENTION

Le domaine technique de l'invention est celui des objets connectés. Plus précisément, l'invention concerne un dispositif connecté utile dans le domaine du suivi comportemental des individus de façon à offrir des moyens d'aide et de surveillance dans des situations compliquées auxquelles les individus ou leurs proches sont confrontés dans la vie courante.

Ainsi, l'invention a pour objet un dispositif portable sur la tête, comme une lunette connectée intelligente, utile pour le suivi du comportement d'un individu et éventuellement son alerte ainsi que les moyens techniques pour sa mise en oeuvre en fonction de ses diverses applications.

ÉTAT DE LA TECHNIQUE

Il est connu de l'état de l'art des dispositifs électroniques portables capables de fournir des informations sur le comportement d'un individu, soit à l'individu lui-même, soit à des tierces personnes, comme des services de secours, et qui peuvent être sauvegardées sur des serveurs personnels ou communautaires. Il s'agit généralement de boîtiers se présentant sous la forme de bracelet et qui peuvent aussi être intégrés dans une montre. A l'origine ces dispositifs étaient souvent destinés à des personnes malades et dont il fallait surveiller le comportement à des fins médicales, généralement en rapport avec des problèmes cardiaques. Ces dispositifs ont évolué et se sont démocratisés notamment pour toucher le quotidien des individus. Ainsi, on connait dans l'art antérieur des dispositifs portables susceptibles de fournir des informations approximatives sur l'activité d'un individu. Ces trackers d'activité sont capables d'enregistrer les déplacements d'un individu et de fournir des données sur le nombre de pas, les distances parcourues, les calories consommées, etc.

Un inconvénient majeur de ces dispositifs est qu'ils n'offrent pas une vision permanente de la situation puisque, soit il faut consulter le boîtier, soit un serveur pour avoir accès en temps réel aux données recueillies et aux alertes qui pourraient être générées.

En outre, un autre inconvénient de ces dispositifs et que les informations sont mises à disposition à l'utilisateur sans aucune analyse, ni interprétation.

On connait aussi dans l'art antérieur des lunettes connectées dont les verres constituent un écran d'affichage de données disponibles sur un téléphone portable ou tout autre terminal, comme des appels téléphoniques, des SMS, des connexions aux réseau sociaux, etc. Aujourd'hui ces lunettes s'orientent davantage vers des applications ludiques, type jeu avec des réalités augmentées.

Il existe donc un besoin pour un dispositif portable offrant un accès permanent en temps réel et donc immédiat à un individu sur son comportement et dont le positionnement du dispositif sur l'individu permette d'accéder à des données nouvelles et pertinentes, et de relayer une restitution facile de l'analyse des données notamment grâce à des alertes auprès de l'individu lui-même ou d'autres individus.

Le document US 2009/105605 décrit différents dispositifs de mesure de paramètres physiologiques, parmi lesquels des lunettes pourvues de différents capteurs.

Le document EP 2 061 026 décrit un dispositif de type lunettes, pour détecter un état physique ainsi que des actions délibérées d'un utilisateur, ces actions consistant essentiellement en des mouvements, notamment de la tête.

Le document US 2001/028309 décrit un dispositif de type lunettes comprenant des moyens pour mesurer la réflexion d'un faisceau lumineux émis, par la cornée ou la paupière de l'utilisateur dudit dispositif.

Le document US 2010/134761 décrit un dispositif de type lunettes comportant un émetteur et un récepteur infrarouge, apte à mesurer la vigilance d'un utilisateur portant un tel dispositif.

OBJET DE L'INVENTION

La présente invention vise précisément à offrir un tel dispositif capable de remédier à tout ou partie des inconvénients de l'état de la technique cités ci-dessus.

A cet effet, la présente invention vise un dispositif de suivi comportemental d'un individu **selon le préambule de la revendication 1 et présentant également les caractéristiques de la partie caractérisante de la revendication 1.**

La monture du dispositif objet de l'invention peut-être articulée ou être sous la forme d'un demi-cercle enserrant la tête.

Dans des modes de réalisation particuliers de l'invention, le dispositif comprend en outre au moins un verre monté sur ladite monture et couvrant au moins l'un des yeux de l'individu.

Ainsi, le dispositif est sous la forme d'une paire de lunettes pouvant comprendre des verres adaptés à la vue de l'individu. Les verres peuvent être teintés ou comprendre tout types de traitement optique connu en soi comme par exemple des traitements anti-reflets, anti-ultraviolets, anti-salissure ou anti-rayures. Le dispositif peut aussi comporter des verres sans corrections optiques ni traitements.

Avantageusement, les moyens de mesure de données physiques de l'individu comprennent la mesure des données suivantes :

- le rythme cardiaque,
- la température corporelle,

et la mesure de l'une au moins des données suivantes :

- les déplacements linéaires de la tête de l'individu selon trois axes,
- les mouvements de rotation de la tête de l'individu selon trois axes,
- les mouvements des pupilles et les clignements des yeux,
- l'oxymétrie,
- la pression artérielle,
- la glycémie.

Ainsi, le positionnement du dispositif sur la tête de l'individu permet de mesurer des données particulières comme les mouvements de la tête, les battements de paupières, les mouvements des yeux, la taille de la rétine, les battements du coeur mesurés au niveau des tempes. Ces données confèrent au dispositif de l'invention des avantages considérables par rapport aux dispositifs de l'art antérieur, notamment, comme cela est indiqué ci-après, pour des applications inaccessibles auparavant.

Les moyens de mesure de données physiques relatives à l'oxymétrie et le rythme cardiaque comprennent par exemple un capteur optique composé de LED et photodiodes.

La température corporelle est mesurée par l'intermédiaire d'un capteur de température à proximité ou en contact de la surface de la peau. Le capteur peut être par exemple un thermocouple ou une sonde à résistance de platine.

Les moyens de mesure de données physiques relatives à la pression artérielle comprennent un capteur de pression placé par exemple en contact de la tempe.

Les moyens de mesure de données physiques relatives aux mouvements des pupilles et aux clignements des yeux comprennent un capteur de suivi des pupilles et des mouvements palpébraux, par exemple par ultrason ou par un système de caméras.

Les moyens de mesure de données physiques relatives à la glycémie artérielle comprennent un capteur infrarouge mesurant le taux de glucose dans le sang à travers la peau ou un capteur chimique pour analyser la sueur de l'individu.

Avantageusement, les moyens de mesure de données physiques relatives aux déplacements linéaires et aux mouvements de rotation de la tête de l'individu comprennent un accéléromètre trois axes et un gyroscope trois axes.

Les trois axes mesurés par l'accéléromètre sont généralement : vers l'avant, sur les côtés et en hauteur
Les trois axes mesurés par le gyroscope sont généralement : le roulis, le tangage et le lacet.

Dans des modes de réalisation particuliers de l'invention, le dispositif de l'invention comprend en outre un ou plusieurs moyens de communication ou de mesure choisis parmi :

- des moyens d'envoi des données physiques de l'individu et de leur analyse vers une plateforme de stockage,
- des moyens de mesure, seul ou en combinaison, des données relatives à l'environnement extérieur suivantes : la direction du Nord magnétique, la température extérieure, la pression extérieure, la qualité de l'air, l'indice UV, l'altitude, la position géographique.
- des moyens d'alerte de l'individu ou d'un ou plusieurs autres individus,
- des moyens de stockage et/ou d'administration de médicament comme de l'insuline.

Les moyens de mesure de la température extérieure comprennent un capteur de température pouvant être par exemple un thermocouple ou une sonde à résistance de platine.

Les moyens de mesure de la pression extérieure comprennent un capteur de pression pouvant être par exemple un capteur piezoélectrique.

Les moyens de mesure du Nord magnétique comprennent un compas magnétique.

Les moyens de mesure de la qualité de l'air comprennent un détecteur chimique de particules.

Les moyens de mesure de l'indice UV comprennent une photodiode à UV.

Selon une première forme de mise en oeuvre préférée de l'invention, les moyens de restitution de l'analyse vers l'individu comprennent un ou plusieurs moteurs générant la vibration du dispositif et/ou un signal vibratoire et/ou un son.

Selon une deuxième forme de mise en oeuvre préférée de l'invention, les moyens de restitution de l'analyse vers l'individu comprennent un système lumineux générant un signal visible sur le dispositif dans le champ de vision de l'individu ou directement sur la rétine de l'individu.

Le signal visible sur le dispositif peut être généré sur une partie d'un verre ou sur une partie de la monture du dispositif.

Selon une troisième forme de mise en oeuvre préférée de l'invention, les moyens de restitution de l'analyse vers l'individu comprennent un projecteur générant des images sur le verre du dispositif dans le champ de vision de l'individu ou directement sur la rétine de l'individu.

Ces restitutions constituent des alertes particulièrement utiles dans plusieurs des applications du dispositif de l'invention comme cela apparaîtra ci-après.

Le dispositif de l'invention comprend également avantageusement des moyens de transmission sans fil, comme un module de type Bluetooth® Low Energy pour communiquer sans fil avec un smartphone, une tablette ou une passerelle, également connue sous le nom de « gateway », permettant une transmission des données vers un serveur distant.

Le dispositif de l'invention peut également comprendre une puce à communication en champ proche (anglais « *Near Field Communication* » ou NFC) de type RFID (acronyme anglais de « *Radio Frequency Identification* »).

Le dispositif de l'invention peut être décliné dans les diverses applications qui sont décrites ci-après.

Dans des modes de réalisation particuliers de l'invention, l'analyse des données aboutit à la détection ou à la prédiction de l'endormissement d'un individu au volant d'un véhicule, l'individu portant le dispositif sur la tête.

Les moyens d'analyse des données physiques de l'individu comprennent la comparaison avec des valeurs de référence dite normales de l'individu ou d'un individu ou population témoin concernant le nombre de mouvements palpébraux, l'activité de la pupille, le déplacement de la tête, la température corporelle, le rythme cardiaque, l'oxymétrie, la pression artérielle.

Dans cette application, les moyens de restitution de l'analyse comprennent une connexion entre ledit dispositif et le véhicule piloté par l'individu de façon à exploiter les éléments du véhicule pour réaliser la restitution de l'analyse, comme la vibration du volant, l'affichage d'alerte sur le tableau de bord, l'alerte de personnes sélectionnées par l'individu, tels les passagers du véhicule, l'émission d'alerte sonore ou même l'arrêt du véhicule.

Dans des modes de réalisation particuliers de l'invention, l'analyse des données aboutit à la détection ou à la prédiction d'un comportement inhabituel d'un individu portant le dispositif sur la tête, notamment la mise en danger de l'individu.

Dans des modes de réalisation particuliers de l'invention, l'analyse des données permet la surveillance de la glycémie de l'individu portant le dispositif sur la tête, ledit dispositif comprenant éventuellement en outre des moyens de stockage et/ou d'administration de l'insuline.

Dans des modes de réalisation particuliers de l'invention, le dispositif de l'invention comprend un circuit électronique comportant un microprocesseur et une mémoire informatique stockant un programme informatique comportant les différents algorithmes de traitement et d'analyse des informations décrites précédemment.

Dans des modes de réalisation particuliers de l'invention, l'analyse des données acquises par les capteurs du dispositif est effectuée à distance.

Ainsi, l'analyse à distance peut être effectuée par un smartphone connecté au dispositif, ou par tout autre autre appareil électronique portable comme une tablette, un ordinateur ou une passerelle (« gateway »). Les données peuvent également être transmises à un serveur distant, traité notamment dans l'informatique en nuage, couramment connu sous le nom anglais de « *cloud computing* ».

BREVE DESCRIPTION DES FIGURES

D'autres avantages, buts et caractéristiques particulières de la présente invention ressortiront de la description non limitative qui suit d'exemples de réalisation particuliers des dispositifs objets de la présente invention, en regard des dessins annexés, dans lesquels :

- la figure 1 représente un individu portant un dispositif de suivi comportemental objet de l'invention selon un premier exemple de mode de réalisation ;
- la figure 2 est une vue en perspective du dispositif objet de l'invention en référence à la figure 1 ;
- la figure 3 représente un individu portant un dispositif objet de l'invention selon un deuxième exemple de mode de réalisation ;
- la figure 4 est une vue détaillée du dispositif objet de l'invention en référence à la figure 3 ;
- la figure 5 représente un individu portant un dispositif objet de l'invention selon un troisième exemple de mode de réalisation ;
- la figure 6 est une vue détaillée du dispositif objet de l'invention en référence à la figure 5 ;

- la figure 7 représente un individu portant un dispositif objet de l'invention selon un quatrième exemple de mode de réalisation.

DESCRIPTION DÉTAILLÉE DE L'INVENTION

La présente description est donnée à titre non limitatif, chaque caractéristique d'un mode de réalisation pouvant être combinée à toute autre caractéristique de tout autre mode de réalisation de manière avantageuse.

On note, dès à présent, que les figures ne sont pas à l'échelle.

Exemple d'un mode de réalisation de l'invention : Paire de lunettes de détection de l'endormissement d'un individu au volant d'un véhicule

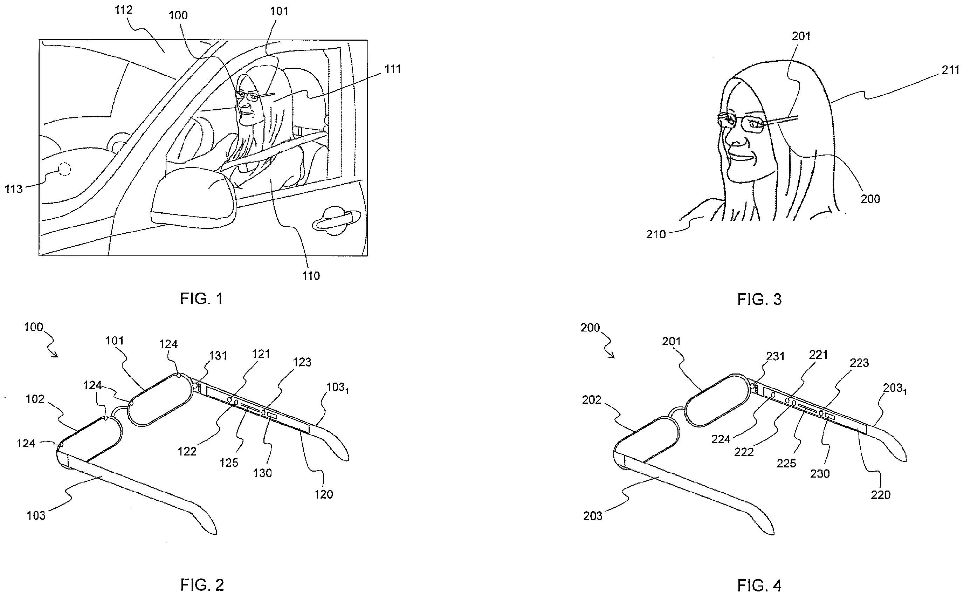

La figure 1 représente un individu 110, couramment appelé conducteur, au volant d'un véhicule 112 comprenant dans le présent exemple non limitatif un accéléromètre 113 et une antenne de communication sans fil. Le conducteur 110 porte sur la tête 111 un dispositif de suivi comportemental selon l'invention.

Le dispositif de suivi comportemental est dans le présent exemple une paire de lunettes connectée 100 comprenant une monture 101 s'étendant de part et d'autre de la tête 111 du conducteur 110. Le dispositif de suivi permet la détection de l'endormissement et l'avertissement du conducteur 110.

La figure 2 représente une vue en perspective de la paire de lunettes 100.

La monture 101 articulée comprend une face 102 et deux branches 103 maintenues à la face 102 par l'intermédiaire de charnières.

La paire de lunettes 100 comprend une carte électronique 120 logée dans le présent exemple non limitatif de l'invention dans la branche droite $103_1$ de la monture 101. La carte électronique 120 reliée à une batterie également logée dans la branche droite $103_1$ comprend un processeur et une mémoire stockant un programme informatique.

Dans cette application, la carte électronique 120, comprend un accéléromètre 121 permettant de mesurer les déplacements linéaires de la tête 111 selon trois axes : dans le sens du déplacement du véhicule 112, dit axe x, dans le sens latéral au déplacement du véhicule 112, dit axe y et dans le sens vertical, dit axe z.

Il convient de souligner que l'accéléromètre 121 mesure également les accélérations liées au déplacement du véhicule 112. Afin de considérer uniquement les déplacements de la tête 111, il convient de soustraire les déplacements du véhicule 112 afin d'obtenir les déplacements de la tête 111 dans le référentiel du véhicule 112. A cet effet, la carte électronique communique par l'intermédiaire d'une antenne 125 de communication sans fil avec l'accéléromètre 113 permettant de mesurer les déplacements du véhicule 112. L'antenne 125 est dans le présent exemple non limitatif de l'invention de type Bluetooth® Low Energy.

Dans le cas où le véhicule 112 ne comprend pas d'accéléromètre 113 permettant de mesurer les déplacements du véhicule 112, un traitement des données acquises par l'accéléromètre 121 de la paire de lunettes 100 permet de décorreler les données liées spécifiquement aux déplacements de la tête 111 de l'individu par rapport au véhicule 112.

La carte électronique 120 comprend également un gyroscope trois axes 122 permettant de mesurer les mouvements de rotation de la tête selon trois axes : le roulis, le tangage et le lacet.

Dans le présent exemple non limitatif de l'invention, la carte électronique 120 comprend également un capteur optique 123 composé de différentes diodes électroluminescentes LED (pour l'anglais « *Light Emitting Diode* ») et d'une photodiode permettant de mesurer le rythme cardiaque et l'oxymétrie du conducteur 110, et un capteur thermique permettant de mesurer la température à proximité de la surface de la peau du conducteur 110.

Afin de mesurer le clignement des yeux, la carte électronique 120 est reliée à un capteur de suivi des pupilles qui formé dans le présent exemple par un système de deux caméras 124 par oeil. Le capteur de suivi peut également être sous la forme d'un capteur à l'utrasons.

Dans une variante de ce mode de réalisation particulier de l'invention, la carte électronique 120 peut également suivre les mouvements des pupilles.

Les données acquises par les différents capteurs de la paire de lunettes 100 sont analysées en temps réel par l'intermédiaire du programme informatique de la carte électronique 120.

L'analyse des données permet de repérer un comportement inhabituel caractéristique de l'endormissement du conducteur.

Ce comportement inhabituel se traduit généralement par un ralentissement du rythme cardiaque, une modification du rythme du clignement des paupières, et une modification du port de la tête, comme une micro-chute.

L'analyse de ces données éventuellement en combinaison avec l'oxymétrie, la température du conducteur 110 et la pression artérielle permet de détecter l'endormissement du conducteur 110.

Il s'agit d'analyser ces données en les comparant avec des valeurs de référence. Avantageusement ces valeurs de référence proviennent du conducteur lui-même en condition de veille normale. Ces valeurs de référence auront été enregistrées, sous la forme d'un étalonnage, lors de l'acquisition du dispositif par le conducteur. Cet étalonnage vient alors en remplacement de valeurs préenregistrées déterminées par exemple sur un panel d'individus dont le comportement au volant aura été analysé via la mesure de mouvements palpébraux, l'activité de la pupille, le déplacement de la tête, la température corporelle, le rythme cardiaque, l'oxymétrie, la pression artérielle.

Ces données confèrent au dispositif de l'invention des avantages considérables par rapport aux dispositifs de l'art antérieur, car ils prennent en compte le comportement personnel du conducteur et pas uniquement des variations de conduite.

Le but de l'algorithme d'analyse présent dans le programme informatique est de détecter au plus tôt l'endormissement du conducteur 110, si possible en détectant des signes avant-coureurs de l'endormissement du conducteur 110.

Dès lors que l'endormissement du conducteur a été détecté, la paire de lunettes 100 émet un signal au conducteur 110 pour le maintenir éveillé, ou le réveiller si nécessaire.

Parmi les moyens de restitution en temps réel de l'analyse vers le conducteur 110, il est envisagé divers signaux qui constituent aussi des moyens d'alertes, lesquels peuvent aller crescendo en fonction de l'analyse. Il peut s'agir de vibrations de la monture 101 générées par des moteurs 130 logés dans la paire de lunettes 100.

Il peut aussi s'agir d'alarmes sonores ou visuelles sous la forme de simples spots lumineux par l'intermédiaire d'une diode électroluminescente LED 131 ou de messages plus instructifs projetés dans le champ de vision du conducteur 110 ou directement sur sa rétine ou encore sur les verres.

Dans cette application, la paire de lunettes 100 interagit par tout système de communication avec le véhicule 112 de façon à exploiter les éléments du véhicule 112 pour réaliser la restitution de l'analyse, comme la vibration du volant, l'affichage d'alerte sur le tableau de bord, l'émission d'alerte sonore ou même l'arrêt du véhicule.

Il convient de souligner que le module de type Bluetooth® Low Energy de la carte électronique 120 peut également communiquer sans fil avec un smartphone ou une tablette, pouvant être reliés avec un serveur distant.

Enfin, il est avantageux de prévoir la communication de la paire de lunettes 100 avec une boîte noire à bord du véhicule 112 ou une plateforme de stockage, de façon à pouvoir récupérer les données en cas d'accident.

Dans une variante de ce mode de réalisation de l'invention, la paire de lunettes 100 comprend également une caméra permettant de regarder l'environnement du conducteur. Avec cette caméra, il est ainsi possible de détecter et d'analyser un panneau de signalisation routière, comme par exemple indiquant la hauteur d'un obstacle sous lequel ne passerait pas le véhicule 112.

Autre exemple d'un mode de réalisation de l'invention : paire de lunettes d'exploration de l'activité d'un individu, notamment dépendant

La figure 3 représente, dans le présent exemple non limitatif de l'invention, un individu 210, âgé, dépendant et atteint par exemple de la maladie d'Alzeihmer ou d'autres troubles privant l'individu 210 de repères ou de mémoire. L'individu 210 porte sur sa tête 211, un dispositif de suivi comportemental, formé par une paire de lunettes 200.

La paire de lunettes connectée 200, illustrée en figure 4, comprenant une monture 201 s'étendant de part et d'autre de la tête 211, permet le suivi de l'activité de l'individu 210 et notamment la détection d'un problème, comme par exemple une chute, survenant ou étant sur le point de survenir.

La monture 201 articulée comprend une face 202 et deux branches 203 maintenues à la face 202 par l'intermédiaire de charnières.

La paire de lunettes 200 comprend une carte électronique 220 logée dans le présent exemple non limitatif de l'invention dans la branche droite $203_1$ de la monture 201. La carte électronique 220 reliée à une batterie également logée dans la branche droite $203_1$ comprend un processeur et une mémoire stockant un programme informatique.

Dans cette application, la carte électronique 220, comprend un accéléromètre 221 permettant de mesurer les déplacements linéaires de la tête 211 selon trois axes : dans le sens du déplacement de l'individu 210, dit axe x, dans le sens latéral au déplacement de l'individu 210, dit axe y et dans le sens vertical, dit axe z.

La carte électronique 220 comprend également un gyroscope trois axes 222 permettant de mesurer les mouvements de rotation de la tête selon trois axes : le roulis, le tangage et le lacet.

Dans le présent exemple non limitatif de l'invention, la carte électronique 220 comprend également un capteur optique 223 composé de différentes diodes électroluminescentes LED (pour l'anglais « *Light Emitting Diode* ») et d'une photodiode permettant de mesurer le rythme cardiaque et l'oxymétrie de l'individu 210, et un capteur thermique permettant de mesurer la température à proximité de la surface de la peau de l'individu 210.

La carte électronique 220 est reliée à un capteur 224 permettant de mesurer la pression artérielle de l'individu 210. Le capteur 224 peut être formé par un coussinet gonflable logé dans chaque des branches 203 de la paire de lunettes 200. Chaque coussinet gonflable est ainsi apte à venir en contact de chaque tempe de l'individu. Un capteur de pression, comme par exemple une jauge de contrainte, est inclue dans chaque coussinet et permet de mesurer la pression artérielle de l'individu 210.

Les données acquises par les différents capteurs de la paire de lunettes 200 sont analysées en temps réel par l'intermédiaire du programme informatique de la carte électronique 220.

L'analyse des données permet de repérer un comportement inhabituel de l'individu 210 comme :

- une période d'inactivité anormalement longue ;
- un choc ou une chute de l'individu 210 ;
- un accident ischémique transitoire (acronyme AIT) ; ou
- un accident vasculaire cérébral (acronyme AVC).

Par exemple, l'accident vasculaire cérébral peut être détecté par une modification au niveau du rythme cardiaque, de la pression artérielle et au niveau de l'oxymétrie.

Il s'agit d'analyser ces données en les comparant avec des valeurs de référence. Avantageusement ces valeurs de référence proviennent de l'individu lui-même en condition standard. Ces valeurs de référence ont été enregistrées, sous la forme d'un étalonnage, lors de l'acquisition du dispositif par l'individu, en effectuant des mouvements définis comme par exemple s'assoir et se lever d'une chaise. Cet étalonnage vient alors en remplacement de valeurs préenregistrées déterminées par exemple sur un panel d'individus dont le comportement aura été analysé via la mesure de mouvements palpébraux, l'activité de la pupille, le déplacement de la tête, la température corporelle, le rythme cardiaque, l'oxymétrie, la pression artérielle.

Ces données confèrent au dispositif de l'invention des avantages considérables par rapport aux dispositifs de l'art antérieur, car ils prennent en compte le comportement personnel de l'individu.

Notamment dans le cas de la détection d'une chute ou d'un AVC, le but de l'algorithme d'analyse présent dans le programme informatique est de détecter le comportement inhabituel au plus tôt, si possible en détectant des signes avant-coureurs liés à la chute ou à l'AVC.

L'analyse peut également permettre de détecter un surmenage ou un stress physique de l'individu 210.

Dès lors que le comportement inhabituel a été détecté, la paire de lunettes 200 émet un signal à l'individu 210.

Parmi les moyens de restitution en temps réel de l'analyse vers l'individu 210, il est envisagé divers signaux qui constituent aussi des moyens d'alertes codées en fonction de l'analyse. Il peut s'agir de vibrations de la monture 201 générées par des moteurs 230 logés dans la paire de lunettes 200.

Il peut aussi s'agir d'alarmes sonores ou visuelles sous la forme de simples spots lumineux par l'intermédiaire d'une diode électroluminescente LED 231 ou de messages plus instructifs projetés dans le champ de vision de l'individu 210 ou directement sur sa rétine ou encore sur les verres.

La carte électronique 210 comprend en outre un module 225 de type Bluetooth® Low Energy pour communiquer sans fil avec un smartphone, une tablette ou une passerelle (« gateway ») portée par l'individu 210.

Il convient de souligner que la passerelle peut être sous la forme d'un bracelet ou attaché à la ceinture de l'individu 210.

Enfin, il est avantageux de prévoir la communication de la paire de lunettes 200 avec un ou plusieurs individus à distance, comme un ou plusieurs parents, service de surveillance ou de santé afin de les alerter qu'un comportement inhabituel est survenu sur l'individu 210.

Il convient de souligner que la paire de lunettes 200 peut également permettre de détecter un comportement inhabituel lié à un éloignement anormal du porteur de la paire de lunettes 200 par rapport à un lieu, par exemple son domicile ou son lieu de soin.

A cet effet, le passage de la paire de lunettes 200 devant une balise placée par exemple au niveau de la porte d'entrée du domicile, active la balise qui identifie la paire de lunettes 200 par l'intermédiaire du module 221 Bluetooth®, et horodate le passage. La balise est reliée à une centrale de surveillance qui analyse les données des différentes balises placées au domicile ou à proximité du domicile afin d'identifier un comportement inhabituel de l'individu 210.

Dans une variante de ce mode de réalisation de l'invention, la paire de lunettes 200 est identifiée par l'intermédiaire d'une puce RFID logée dans la monture 201.

Ainsi, l'individu 210 peut être suivi grâce à sa paire de lunettes 200 portée sur le visage.

Autre exemple d'un mode de réalisation de l'invention : paire de lunettes pour la surveillance de la glycémie

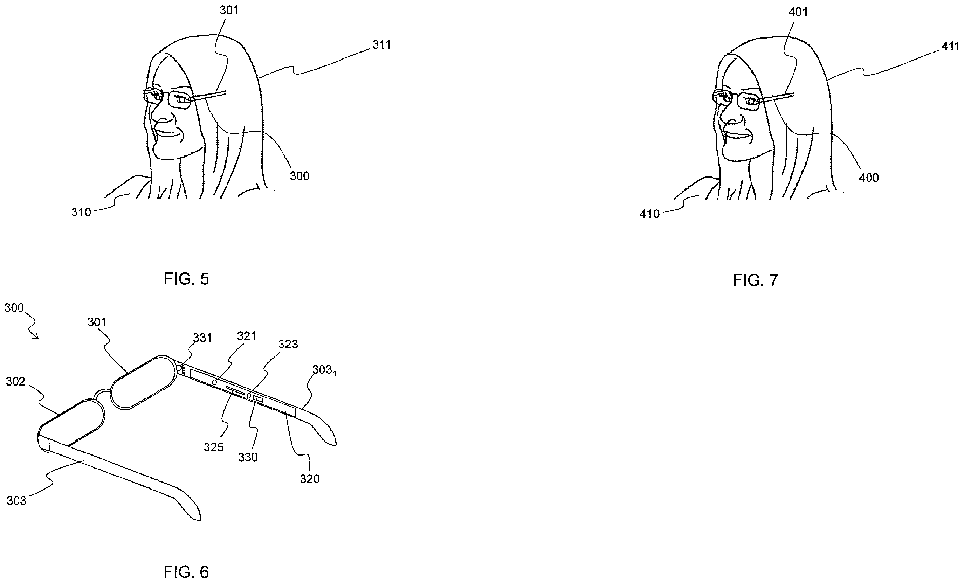

La figure 5 représente, dans le présent exemple non limitatif de l'invention, un individu 310 atteint d'un diabète. L'individu 310 porte une paire de lunettes connectée 300 selon l'invention sur sa tête 311.

La paire de lunettes 300, illustrée en figure 6, comprenant une monture 301 s'étendant de part et d'autre de la tête 311, permet la surveillance de la glycémie de l'individu 310 et notamment la détection d'un problème, comme par exemple une chute, survenant ou étant sur le point de survenir.

La monture 301 articulée comprend une face 302 et deux branches 303 maintenues à la face 302 par l'intermédiaire de charnières.

La paire de lunettes 300 comprend une carte électronique 320 logée dans le présent exemple non limitatif de l'invention dans la branche droite 303$_1$ de la monture 301. La carte électronique 320 reliée à une batterie également logée dans la branche droite 303$_1$ comprend un processeur et une mémoire stockant un programme informatique.

Dans cette application, la carte électronique 320 comprend un capteur de glycémie 321 pouvant être un capteur infrarouge mesurant le taux de glucose dans le sang à travers la peau ou un capteur chimique pour analyser la sueur de l'individu 310.

La carte électronique 320 comprend également un capteur optique 323 composé de différentes diodes électroluminescentes LED (pour l'anglais « *Light Emitting Diode* ») et d'une photodiode permettant de mesurer le rythme cardiaque et l'oxymétrie de l'individu 310, et un capteur thermique permettant de mesurer la température à proximité de la surface de la peau de l'individu 310.

Les données acquises par les différents capteurs de la paire de lunettes 300 sont analysées en temps réel par l'intermédiaire du programme informatique de la carte électronique 320.

L'analyse des données permet de repérer une valeur anormale de glycémie, voire un dépassement de seuil pour le rythme cardiaque, l'oxymétrie, la pression artérielle ou la température de l'individu 310.

Il s'agit d'analyser ces données en les comparant avec des valeurs de référence. Avantageusement ces valeurs de référence proviennent de l'individu 310 lui-même et sont acquises par un traitement statistique. Les valeurs de référence peuvent également provenir d'un individu témoin concernant le taux de glucose.

Dès lors que le comportement inhabituel a été détecté, la paire de lunettes 300 émet un signal à l'individu 310.

Parmi les moyens de restitution en temps réel de l'analyse vers l'individu 310, il est envisagé divers signaux qui constituent aussi des moyens d'alertes codées en fonction de l'analyse. Il peut s'agir de vibrations de la monture 301 générées par des moteurs 330 logés dans la paire de lunettes 300.

Il peut aussi s'agir d'alarmes sonores ou visuelles sous la forme de simples spots lumineux par l'intermédiaire d'une diode électroluminescente LED 331 ou de messages plus instructifs projetés dans le champ de vision de l'individu 310 ou directement sur sa rétine ou encore sur les verres.

La paire de lunettes 300 comprend également un stockage d'un produit comme l'insuline qui peut être administré automatiquement à l'individu 310 dès lors qu'un taux anormal de glycémie est détecté, par l'intermédiaire d'un dispositif mécanique 340 logé à l'intérieur d'une branche 303 en regard d'une tempe de l'individu 310. A cet effet, le dispositif mécanique 340 comprend une ou plusieurs micro-aiguilles 341 qui entrent en contact avec la peau de l'individu 310.

Selon une forme particulière de réalisation de cette application, les moyens de stockage et/ou d'administration de l'insuline comprennent un manchon de type patch disposé sur la monture.

La carte électronique 310 comprend en outre un module 325 de type Bluetooth® Low Energy pour communiquer sans fil avec un smartphone ou une tablette, pouvant être relié à un serveur distant.

Dans cette application, les moyens de restitution de l'analyse comprennent la fourniture à l'individu muni du dispositif ou à un ou plusieurs parents, service de surveillance ou de santé, d'informations sur les variations de la glycémie en fonction de l'alimentation, de l'activité physique, d'une perte ou d'une augmentation du poids pour mieux contrôler un diabète.

Autre exemple d'un mode de réalisation de l'invention : paire de lunettes pour l'exploration de l'activité de l'individu

La figure 7 représente un individu 410 effectuant une activité physique d'extérieur ou en salle. L'individu 410 porte une paire de lunettes 400 selon l'invention sur sa tête 411.

La paire de lunettes connectée 400 est utile pour l'exploration de l'activité de l'individu 410.

Dans cette application, la paire de lunettes 400 comprend :

- des moyens de mesure de données physiques de l'individu,
- des moyens d'analyse desdites données en temps réel,
- des moyens de restitution de ladite analyse vers l'individu,
- des moyens d'envoi desdites données et de leur analyse vers une plateforme de stockage.

Les moyens de mesure de données physiques de l'individu comprennent la mesure des données suivantes :

- les déplacements linéaires de la tête de l'individu selon trois axes : vers l'avant, sur les côtés et en hauteur,
- les mouvements de rotation de la tête de l'individu selon trois axes : le roulis, le tangage et le lacet,
- le rythme cardiaque, l'oxymétrie, la pression artérielle,
- la température corporelle,
- la direction du Nord magnétique,
- la température extérieure,
- la pression extérieure,
- la qualité de l'air

- l'indice UV.

Les moyens d'analyse des données physiques de l'individu comprennent la comparaison avec des valeurs de référence dite normales de l'individu ou d'un individu témoin concernant son emplacement géographique, son déplacement, comme le repos, la marche, la course, l'altitude ou la profondeur.

Dans cette application, les moyens de restitution de l'analyse comprennent la fourniture d'informations sur les vitesses, les distances, les calories, les alertes par rapport à des seuils ou des conditions particulières de l'environnement, les comparaisons par rapport à des restitutions passées.

Il convient de souligner que ce dispositif permet d'éviter via une alerte les risques de surmenage physique pouvant donner lieu à un accident, comme par exemple un accident cardiovasculaire.

Dans des variantes des modes de réalisation de l'invention, l'analyse des données acquises par les capteurs logés dans la paire de lunettes est effectuée par l'intermédiaire d'une application traitée à distance par le smartphone ou par la tablette, pouvant communiquer avec un serveur distant stockant des données statistiques relatives à l'utilisateur portant la paire de lunettes ou relatives à un panel d'utilisateurs.

Dans des variantes des modes de réalisation de l'invention, l'étalonnage des données est mise à jour régulièrement par un traitement automatique des données, effectuant ainsi un auto-apprentissage des données acquises de l'individu.

Dans des variantes des modes de réalisation de l'invention, la paire de lunettes comprend également un module de géolocalisation par satellites, comme par exemple un module GPS (acronyme anglais de « *Global Positioning System* »)*.*

Dans des variantes des modes de réalisation de l'invention, la paire de lunettes comprend également un module de communication au standard GSM (acronyme anglais de « *Global System for Mobile communications* ») permettant de communiquer les données recueillies par les capteurs de la paire de lunettes.

Dans des variantes des modes de réalisation de l'invention, la paire de lunettes échange des informations avec un bracelet connecté ou avec une semelle connectée. Le bracelet ou la semelle pouvant comprendre un ou plusieurs capteurs de type similaire ou non à ceux du dispositif de suivi comportemental.

On décrit maintenant des variantes et précisions par rapport aux formes de réalisation décrites ci-dessus.

Plus précisément, il est décrit ci-après un dispositif susceptible d'être porté sur la tête d'un individu, à proximité des yeux, et comportant des moyens aptes à mesurer :

- des paramètres de comportement dudit individu, à savoir, notamment :

  - l'endormissement,
  - un clignement (battement) de paupière involontaire,
  - un clin d'oeil volontaire,
  - la position du dispositif sur la tête de l'individu,

L'invention est applicable aux situations où l'individu réalise une opération qui requiert son attention quasi permanente, telle que par exemple la conduite d'un véhicule, une machine fixe (presse, scie, etc.).

Il existe à ce jour de tels dispositifs, mais qui ne donnent pas entièrement satisfaction.

En effet, ces dispositifs connus ne permettent pas de concilier les impératifs suivants, qui se révèlent contradictoires :

- mesures de plusieurs paramètres distincts
- traitement poussé des données mesurées
- interactivité avec l'individu
- grande autonomie
- robustesse
- fiabilité
- aspect discret, sans affecter l'esthétique des lunettes sur lesquelles le dispositif est incorporé ou rapporté.

L'invention remédie à cette situation et propose un dispositif qui présente tous les avantages ci-dessus.

De plus, le dispositif présente l'avantage de pouvoir être associé à une paire de lunettes de protection et/ou de vue et/ou de soleil, soit en étant :

- intégré dans une structure de lunettes conçues à cet effet ;
- rapporté et fixé sur des lunettes "ordinaires".

Précisément, lesdits moyens de détection incluent :

- au moins un moyen émetteur de lumière, suivant une direction dirigée vers au moins un oeil, lorsque le dispositif est porté ;
- au moins un moyen capteur de la lumière réfléchie par ledit oeil.

Avantageusement, pour chaque oeil, sont prévus un capteur et deux moyens émetteurs, ces deux derniers étant distants de part et d'autre d'une direction sensiblement horizontale, correspondant à l'axe optique de l'individu lorsque le dispositif est porté.

De préférence, les émetteurs de lumière et le capteur optique sont du type infra rouge.

Selon une forme avantageuse, les moyens de traitement et de comparaison comportent des moyens de détection de paramètres représentatifs de l'endormissement comportant :

- un filtre du type « Butterworth »
- un module de calcul de la dérivée du signal, dont les deux sorties sont respectivement reliées à :

    1. un élément de détection de pic de signal, relié à un moyen de stockage de données, lui-même relié à un comparateur binaire, débouchant sur deux éléments respectivement de comptage de la durée d'ouverture / de fermeture de la paupière ;
    2. un élément de calcul de la variance de la dérivée, relié à un premier comparateur avec un seuil, débouchant sur un compteur, lui-même relié à un élément apte à rechercher le dernier pic de signal (dans le moyen de stockage).

Lesdits moyens de traitement et comparaison comportent des moyens de détection de clignement de paupière involontaire, incluant successivement :

- un filtre du type « Butterworth »
- un élément de calibration, relié à un moyen de stockage des paramètres de l'algorithme de traitement
- un moyen de détection de pic de signal
- un élément comparatif de la valeur du pic à une valeur étalon / seuil
- un élément de confirmation de pic, représentatif d'un clignement de paupière
- un organe de traitement des données relié à un moyen de stockage.

De plus, un élément de calcul prélève les données issues du filtre pour les injecter, après dérivation, dans l'organe de traitement.

Lesdits moyens de traitement et comparaison comportent des moyens de détection de clin d'oeil (volontaire) incluant :

- un filtre du type « Butterworth »

    - un élément de calibration (pour tenir compte des particularités de l'individu porteur)
    - un moyen de détection de pic de signal
    - un élément comparatif de la valeur du pic à une valeur étalon / seuil
    - un élément de confirmation de pic, représentatif d'un clin d'oeil (volontaire).

Le dispositif comporte en outre une source d'alimentation autonome (telle qu'une pile ou batterie), et des moyens de contrôle et régulation de la consommation électrique de ladite source.

De plus, le dispositif comporte des moyens de détection et de mesure de sa position sur la tête de l'individu.

De plus, le dispositif comporte des moyens de transmission permettant de configurer et mettre à jour l'algorithme et les données stockées (seuils, calibration, etc.)

L'invention sera bien comprise à la lumière de la description qui suit de rapportant aux dessins suivants, représentant des exemples non limitatifs de formes de réalisation.

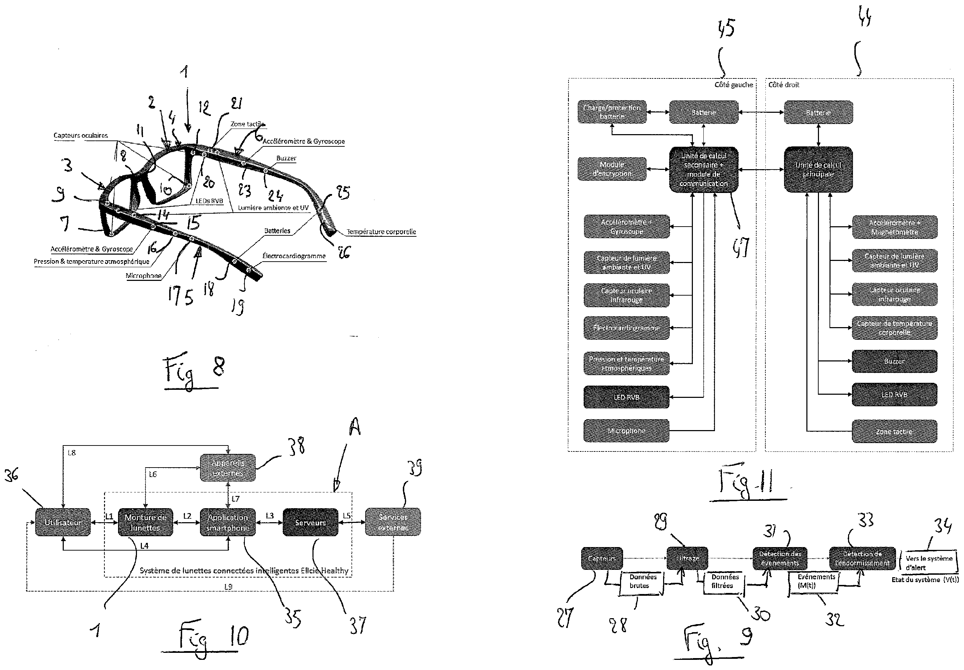

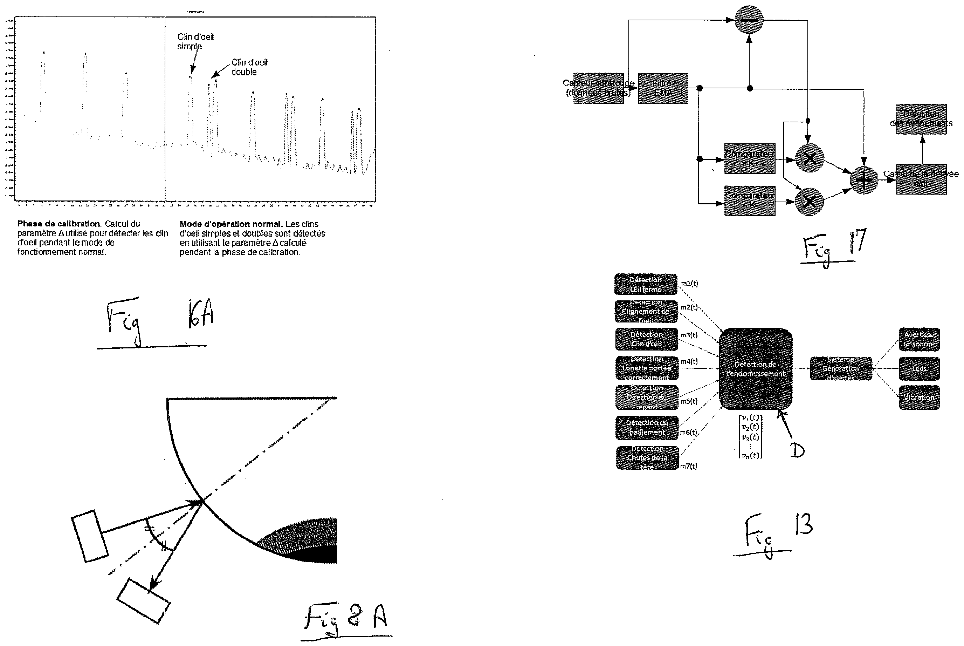

- La figure 8 est une vue en perspective arrière d'une paire de lunettes pourvue du dispositif de l'invention ;

    - La figure 8A est un schéma simplifié montrant la disposition des capteurs optiques par rapport à l'oeil.

- La figure 9 montre de manière schématique le cheminement linéaires des données de mesures ;

- La figure 10 est un organigramme schématique des principaux blocs fonctionnels du dispositif et leur liaison ;
- La figure 11 est un synoptique des différents éléments constitutifs du dispositif de l'invention et leur liaison fonctionnelle sur les deux branches d'une paire de lunettes ;
- La figure 12 est un organigramme général du traitement des mesures pour la détection de l'endormissement de l'individu porteur du dispositif ;

- La figure 13 est un organigramme du traitement des mesures pour la détection de clignement de paupière (involontaire) ;
- Les figures 13A et 13B montrent des courbes de variation temporelle de la variance du signal issu des capteurs optiques ;

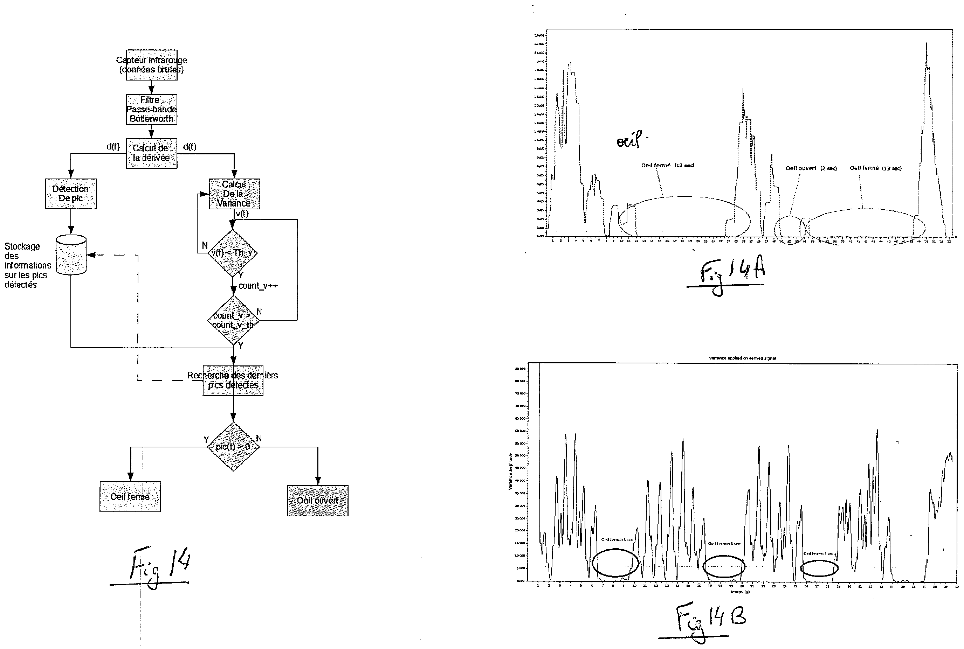

- La figure 14 est un organigramme du traitement des mesures pour la détection de clin d'œil (volontaire) ;
- Les figures 14A et 14B montrent des exemples de variation temporelle des variances de signaux issus des capteurs, après traitement ;

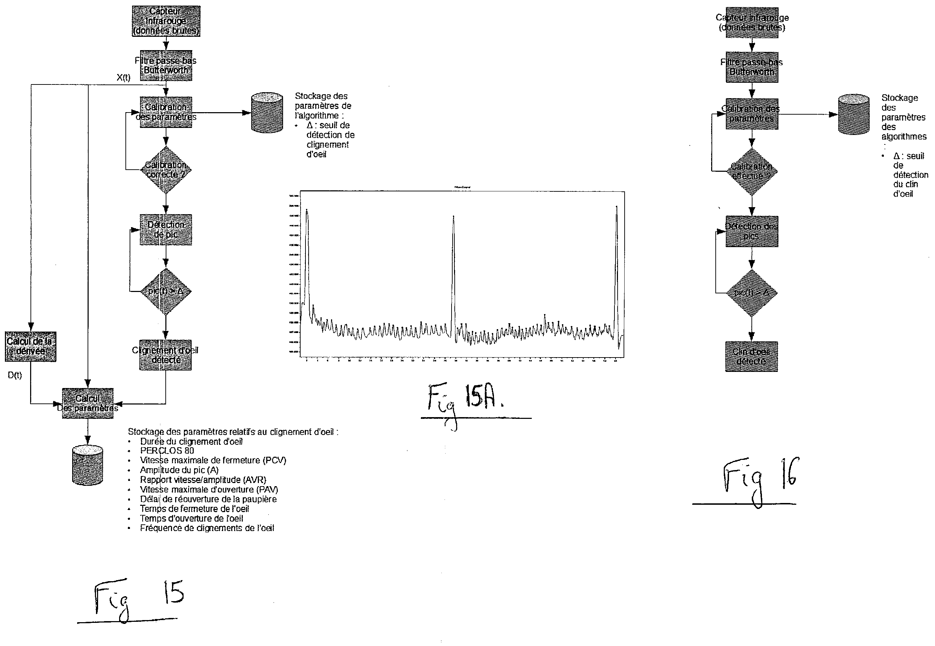

- La figure 15 est un organigramme du traitement des mesures pour la détection d'un battement de paupière involontaire de l'individu porteur ;
- La figure 15A montre une courbe de variation temporelle de la variance du signal, issu des mesures réalisées (organigramme de la figure 15) ;
- La figure 16 illustre un graphe de variation dans le temps (en secondes) de la variance (au sens mathématique) de l'intensité du signal lumineux reçu par le capteur optique (pour un oeil) ;

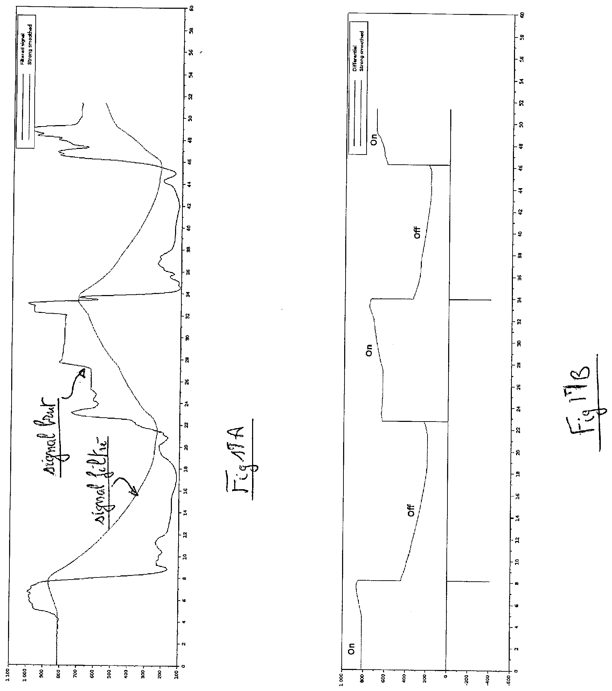

- La figure 17 est un organigramme du processus de détection de port ou d'absence de port des lunettes par l'individu ;
- La figure 17A montre deux courbes de variation temporelle du signal respectivement brut et filtré ;
- La figure 17B montre une courbe de variation temporelle du signal filtré de la figure 10A, après dérivation ;

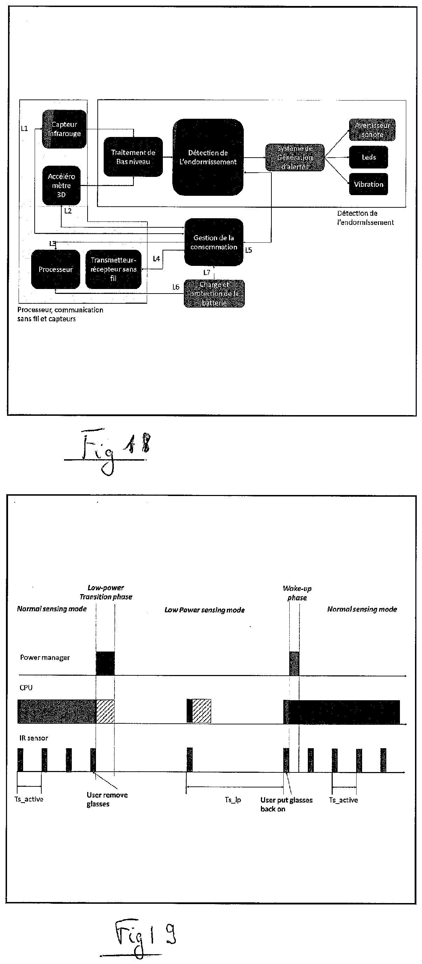

- La figure 18 montre un organigramme fonctionnel des moyens de régulation et de contrôle de la consommation électrique ;
- La figure 19 montre trois diagrammes temporels montrant les consommations et périodes actives électriquement, respectivement des capteurs et du processeur, et les phases de démarrage.

La figure 8 montre en perspective un exemple de réalisation de l'invention, comprenant une paire de lunettes 1, de type connu en soi, incluant une monture 2 formée de deux anneaux 3 et 4 entourant chacune un verre de vision ou de protection. Deux branches latérales 5 et 6 sont articulées sur chaque anneau par deux charnières.

Selon l'invention, sont prévus les organes, éléments et moyens suivants :

- sur l'anneau de gauche 3 : deux émetteurs de lumière infrarouge respectivement inférieur 7 et supérieur 8 et un capteur optique infrarouge 9 (au niveau de la charnière) ;

- sur l'anneau de droite 4 : deux émetteurs de lumière infrarouge respectivement inférieur 10 (au coin inférieur gauche de l'anneau) et supérieur 11 (au niveau de la charnière), et un capteur optique infrarouge 12 (au niveau de la charnière) ;

- sur la branche gauche 5 (en partant de la charnière) :

   * une LED RGB 13
   * un capteur de lumière ambiante 14

   • un accéléromètre et magnétomètre 15
   • un capteur de pression atmosphérique et de température extérieure 16
   • un microphone 17
   • des premières batteries 18
   • un capteur pour réaliser un électrocardiogramme 19

- sur la branche droite 6 :

   • une LED RGB 20
   • une zone tactile 21
   • un capteur 22 de lumière ambiante
   • un accéléromètre et gyroscope 23
   • un émetteur 24 de son vibrant (dénommé « buzzer » en anglais)

- des secondes batteries 25
- un capteur de température corporelle 26.

La détection de l'endormissement, clignement (battement) de paupière, clin d'oeil, est basée sur les mesures de la lumière issue des émetteurs LED réfléchie par l'oeil.

Les émetteurs LED 7, 8 et 10, 11 d'une part, et les capteurs optiques 9 et 12 sont disposés et présentent des axes optiques ad hoc, pour optimiser les mesures de la lumière réfléchie par l'oeil. La figure 8A montre schématiquement l'orientation des capteurs et des émetteurs qui font face à l'oeil, afin de concentrer la lumière réfléchie vers le capteur.

Les accéléromètres 15 et 23 sont placés de part et d'autre de la tête par rapport à son axe de rotation vertical, pour calculer sa rotation à l'aide d'un modèle cinétique de type connu.

Les capteurs de lumière ambiante 14 et 22 sont placés en partie haute des branches 5 et 6, de chaque côté de la tête, pour réduire l'impact de l'ombre de cette dernière et permettre de calculer précisément l'intensité de la lumière ambiante.

Le capteur de température extérieure 26 est disposé sur l'extérieur de la branche pour minimiser l'influence de la température corporelle de l'individu.

Les électrodes de mesure pour réaliser un électrocardiogramme sont placées derrière les oreilles, à l'endroit où l'extrémité distale de la branche est pressée contre le mastoïde (os temporal) et assurer un bon contact électrique.

La figure 9 représente de manière très schématique le flux des données brutes 28 issues des capteurs (bloc 27), qui deviennent, après filtrage 29, des données filtrées 30 alimentant la détection d'événements 31, délivrant des événements 32 eux-mêmes alimentant la détection de l'endormissement 33 (ou de clignement de paupière ou clin d'oeil). La sortie du bloc 33 est reliée à des systèmes d'alerte, d'alarme, de stockage, ou autre (bloc 34).

Sur la figure 10, on a représenté de manière schématique les blocs fonctionnels et leur liaisons respectives, au regard du flux de données, à savoir :

- un bloc fonctionnel A (en traits discontinus) représentant l'ensemble des éléments constitutifs du dispositif de l'invention intégrés ou fixés sur les lunettes, et incluant :

    * la monture (2, 5 et 6) de la paire de lunettes 1 (de la figure 8)
    * des applications de téléphone portable 35 relié (L2) à un individu utilisateur/trice 36 et relié aussi (L3) à des serveurs 37 intégrés à la monture de lunettes.

L'individu est apte à agir (liaison L8) sur des appareils externes 38 (décrits plus loin), eux-mêmes reliés (L6) à la paire de lunettes 1. Les serveurs 37 sont reliés (L5) à des services externes 39 (par exemple : service de gestion de flotte de véhicules, services d'assistance dépannage, centres de recherche scientifique).

Le tableau ci-après liste de manière non exhaustive les différentes liaisons et fonctions de la figure 10.

| Lien | Direction | Interface | Données transmises |
|------|-----------|-----------|--------------------|
| L1 | Utilisateur → Lunettes | Capteurs | - Données physiques, physiologiques et environnementales<br>- Gestes captés par la zone tactile<br>- Commande vocale |
|  | Lunettes → Utilisateur | Actionneurs (LEDs RVB et transducteur sonore) | - Signaux lumineux<br>- Signaux sonores |
| L2 | Lunettes → Application smartphone | Liaison sans fil (Bluetooth Low Energy ou similaire) | - Information traitée<br>- Alertes<br>- Données capteur brutes pour étude et apprentissage |
|  | Application smartphone → Lunettes |  | - Configuration<br>- Mises à jour logicielles |

(suite)

| Lien | Direction | Interface | Données transmises |
|------|-----------|-----------|--------------------|
| L3 | Application smartphone → Datacenter | Liaison sans fil (réseau de téléphonie mobile, Wifi...): service web et SMS | - Information traitée<br>- Alertes<br>- Données capteur brutes pour étude et apprentissage |
| | Datacenter → Application smartphone | | - Configuration<br>- Mises à jour logicielles |
| L4 | Application smartphone → Utilisateur | Écran et haut-parleur | - informations sur l'état actuel<br>- Alertes<br>- Conseils<br>- Statistiques |
| | Utilisateur → Application smartphone | Écran tactile et commande vocale | - Configuration<br>- Commandes (démarrage, arrêt...)<br>- Commentaires et demandes de services |
| L5 | Datacenter → Services tiers | Web service | - Alertes<br>- Statistiques |
| | Services tiers → Datacenter | | - Configuration<br>- Information externes |
| L6 | Lunettes → Appareils tiers | Liaison sans fil (Bluetooth Low Energy ou similaire) | - Contrôle d'appareils externes<br>- Données pour traitement externe |
| | Appareils tiers → Lunettes | | - Diffusion d'informations et d'alertes<br>- Données de capteurs externes |
| L7 | Application smartphone → Appareils tiers | Liaison sans fil (Bluetooth Low Energy ou similaire) ou autre selon l'appareil tiers | - Contrôle d'appareils externes<br>- Données pour traitement externe |
| | Appareils tiers → Application smartphone | | - Diffusion d'informations et d'alertes<br>- Données de capteurs externes |

La figure 11 reprend les éléments décrits ci-dessus en regard des figures 8 à 10, en détail et pour préciser leurs liens fonctionnels. Les éléments communs, entre la figure 11 d'une part et les figures 8 à 10 d'autre part, portent donc les mêmes références.

Quatre batteries 180, 181 (branche gauche) et 250, 251 (branche droite) sont prévues, et reliées à un circuit 40 de gestion (contrôle et régulation), lui-même lié à un connecteur 41 apte à être relié à une source de recharge (non représentée) des batteries.

Un circuit 42, dit circuit principal, est relié au circuit de gestion 40, à chacun des capteurs, émetteurs de lumière, et autres moyens de mesures montrés sur la figure 8, et intégrés à la monture de lunettes, et en outre à une antenne 43.

La figure 11 montre les moyens et éléments constitutifs et leur liaisons respectives, distinguant ceux intégrés sur les côtés respectivement droit 44 et gauche 45 de la figure.

Un module de traitement et de mesures est prévu dans chaque couple (3, 5) et (2, 6) branche / anneau porteur de verre. Les deux modules sont reliés l'un à l'autre par des bus série et des lignes d'alimentation électriques.

Le module principal (dit aussi de droite) est destiné principalement au traitement des signaux et extraction d'informations, tandis que le module secondaire (dit aussi de gauche) est destiné principalement à la communication d'une part vers et depuis l'extérieur des lunettes (communication qui est sécurisée par cryptage et/ou vérification de signature de messages), et d'autre part des capteurs d'une branche à l'autre.

L'unité de calcul principale 46 et l'unité de calcul secondaire 47 forment à elles deux un circuit dit principal.

Les programmes et logiciels incorporés dans le dispositif de l'invention permettent les fonctions suivantes :
Les logiciels tournant sur les processeurs intégrés dans la monture sont chargés de :

- Configurer les capteurs et autres périphériques,
- Acquérir les données capteur,
- Filtrer les données acquises,
- Détecter et caractériser des événements à partir des données acquises,
- Calculer le niveau d'endormissement et d'autres indicateurs en temps réel à partir des événements détectés et du profil utilisateur,
- Générer des signaux d'alerte et d'information,
- Répondre aux ordres de l'utilisateur,
- Transmettre des données à travers la liaison sans fil,
- Assurer la sécurité des données
- Optimiser la consommation énergétique (cf. chapitre 8),
- Appliquer les mises à jour reçues via la liaison sans fil.

L'application smartphone est en charge de :

- Appairer la monture et le téléphone de l'utilisateur de manière sécurisée,
- Configurer le logiciel embarqué (sensibilité, intensité des signaux sonores et visuels),
- Sauvegarder et restaurer les paramètres de configuration vers / depuis des serveurs distants,
- Échanger des données de manière bidirectionnelle avec la monture en utilisant une liaison sans fil,
- Échanger des données de manière bidirectionnelle avec des serveurs distants en utilisant toute forme de connexion à l'Internet disponible (protocole http),
- Afficher ou diffuser des signaux d'alerte émis par la monture en utilisant une connexion à l'Internet et/ou un service de messages courts,
- Afficher des statistiques,
- Afficher des conseils pour diminuer les risques encourus par l'utilisateur,
- Récolter les commentaires de l'utilisateur en cas de faux-positif pour aider à affiner les algorithmes
- Récolter les commentaires de l'utilisateur le fonctionnement général du dispositif,
- Afficher les instructions de calibration et d'utilisation,
- Vérifier l'authenticité du logiciel intégré dans le dispositif,
- Piloter la mise à jour du logiciel intégré dans le dispositif

Selon d'autres formes de réalisation :

- Le téléphone portable est remplacé par un autre appareil, distinct des lunettes, conçu et apte à réaliser les fonctions et opérations décrites ci-dessus (par exemple une connexion directe au système informatique du véhicule).
- Il est également possible de concevoir et mettre à disposition un kit de développement permettant à des tiers de développer et distribuer des applications mobiles additionnelles tirant parti des fonctionnalités de la monture.

Les données reçues des capteurs, après traitement, peuvent être envoyées vers des serveurs via Internet ou tout autre moyen d'envoi de données. Lesdits serveurs permettent de :

- Recevoir et stocker de manière sécurisée les données générées par le dispositif de l'invention,
- Recevoir et stocker de manière sécurisée la configuration propre (calibration) à chaque individu,
- Calculer des statistiques concernant un individu particulier ou une population d'individus,
- Se connecter à des services en ligne tiers pour transmettre des alertes ou des données statistiques,
- Recevoir et interpréter des messages d'alerte transmis par service de messages courts, type SMS,
- Envoyer des messages courts, type SMS, à des services tiers pour déclencher une intervention en cas d'urgence,
- Se connecter à des services en ligne tiers pour récupérer des données et les transmette au logiciel intégré dans les lunettes, pour enrichir le traitement du signal ou fournir des informations additionnelles à l'utilisateur (niveau de pollution atmosphérique par exemple),
- Exécuter des algorithmes d'intelligence artificielle et d'optimisation pour adapter les paramètres des algorithmes à chaque individu,
- Gérer le déploiement des mises à jour du logiciel embarqué sur l'ensemble du parc de montures en service.

La figure 13 montre un organigramme général de la fonction de détection d'endormissement.

On voit que cette détection comprend plusieurs mesures de différents paramètres liés :

- d'une part aux mouvements des paupières : oeil fermé, clignement de paupière, clin d'oeil ;

- d'autre part à d'autres paramètres (hors mouvement des paupières) : position des lunettes (portées ou absentes), bâillement, basculement de la tête en avant, direction du regard.

On note l'importance de pouvoir distinguer, une fois détecté la position fermée de l'oeil, entre l'endormissement, un clignement (battement) de paupière involontaire, et un clin d'oeil volontaire.

Le clin d'oeil peut être utilisé comme un signal émis volontairement par l'individu, pour déclencher ou commander un appareil ou une fonction, dans l'environnement immédiat, comme par exemple, pour un individu conduisant un véhicule : ouvrir ou fermer une portière ou le coffre, régler le volume de la radio/musique, etc.

En cas de détection d'un endormissement de l'individu, le dispositif émet des signaux d'alertes, sonores et/ou visuelles, destinés à réveiller l'individu et/ou donner l'alerte à distance.

La figure 14 est un organigramme relatif à la détection de la fermeture prolongée d'un œil.

Les données brutes issues des capteurs optiques sont traitées :

- D'abord par un filtrage 48 du type « Butterworth », qui permet d'atténuer / éliminer les artefacts et les interférences avec l'environnement (lumière, vibrations, etc.) ;
- Ensuite, on calcule 49 la dérivée du signal, qui est injectée :

    * vers un moyen de détection de pic, suivi d'un moyen de stockage

    * vers un moyen de calcul de la variance 50 de la dérivée

    * suivi d'un premier comparateur 51, comparant ladite dérivée avec une valeur de seuil (typiquement comprise entre 8 et 10% de la valeur maximale de la variance) ; si la variance reste en dessous dudit seuil pendant un certain nombre de décomptes du compteur, cela signifie que l'oeil (la paupière) est en position statique, soit ouvert, soit fermé.

    * le comparateur alimente un compteur 52, relié à un élément de recherche 53 du dernier pic de signal (dans le moyen de stockage), ce qui permet de déterminer l'état (ouvert ou fermé) de l'oeil. La dernière mesure de pic est analysée, à l'aide de l'information issue du dernier battement ou clignement de paupière.

Enfin, un second comparateur 54 compare la valeur du pic à un seuil, qui conduit à l'un des états : oeil fermé 55 / oeil ouvert 56.

La figure 14A montre une courbe de variation temporelle (en secondes) de la variance calculée, qui révèlent les périodes respectives de fermeture et d'ouverture de l'oeil. La ligne horizontale en traits discontinus représente le seuil utilisé pour détecter le début d'un événement de fermeture de l'oeil (sans que l'on puisse à ce stade savoir encore s'il s'agit d'un endormissement ou autre).

La figure 14B est une variante de la figure 14A, montrant que les événements de fermeture d'œil ont, dans cet exemple, une durée entre 2 et 3 secondes. La valeur seuil utilisée pour tester le décompte, afin de détecter les événements de fermeture de l'oeil, doivent être réglés en fonction de la durée typique d'événements de fermeture de l'œil.

La variance du signal infrarouge dépend de la dynamique du signal et de la résolution de ce dernier. Les valeurs seuils utilisées pour tester la variance, en vue de déclencher la détection de fermeture de l'oeil, peuvent être réglées à une valeur comprise entre 8% et 10 % de la valeur maximale de la variance, pour réaliser une détection fiable.

Lorsque la variance est inférieure audit seuil, un décompte est déclenché. Si la variance reste sous le seuil pendant un certain temps (nombre de décomptes temporels), alors le dispositif déclenche un événement « fermeture oeil ».

La figure 15 montre un organigramme de cette détermination par le dispositif qui comporte à cette fin :

- un filtre du type « Butterworth »
- un élément de calibration, relié à un moyen de stockage des paramètres de l'algorithme de traitement
- un moyen de détection de pic de signal
- un élément comparatif de la valeur du pic à une valeur étalon / seuil
- un élément confirmation de pic, représentatif d'un clignement de paupière
- un organe de traitement des données relié à un moyen de stockage
- un élément de calcul qui prélève les données issues du filtre pour les injecter, après dérivation, dans l'organe de traitement.

La figure 15A montre une courbe de variations temporelles (en secondes) du signal calibré issu du capteur, qui révèlent un pic très net, représentatif d'un clignement de paupière (involontaire).

La figure 16 montre un organigramme de la fonction de détection de clin d'oeil (volontaire).

La figure 16A illustre un graphe temporel de variation dans le temps (en secondes) de la variance (au sens mathématique) de l'intensité du signal lumineux reçu par le capteur optique (pour un oeil).

La courbe présente des séries successives de pics de faible amplitude (dits petits pics) représentatifs de clignements de paupière involontaires), interrompue par des pics de grande amplitude (représentant chacun un clin d'oeil volontaire).

La détection de l'endormissement fait appel à des calculs de toute combinaison de paramètres suivants :

- Durée du clignement : temps séparant la fermeture et l'ouverture de l'oeil mesurés à la moitié de l'amplitude de déplacement de la paupière. Cette définition permet de caractériser les clignements même en présence de mouvements du regard. La durée du clignement mesurée de cette façon augmente proportionnellement avec le degré de somnolence. La durée typique d'un clignement est comprise entre 300 et 400ms.
- PERCLOS (PERcentage of eye CLOSure - Pourcentage de fermeture de l'oeil) : indicateur couramment utilisé, correspondant à la durée pendant laquelle les paupières sont closes à plus de 80%.
- Amplitude du clignement : distance parcourue par les paupières entre le début du clignement (oeil ouvert) et leur fermeture. La somnolence est corrélée expérimentalement à une baisse de l'amplitude des clignements.
- Vitesse de fermeture et d'ouverture des paupières : vitesse de déplacement maximale atteinte par le bord de la paupière lors de leur fermeture et de leur ouverture, respectivement. Ces vitesses diminuent toutes deux avec l'augmentation de la somnolence.
- Rapport amplitude/vitesse du clignement : défini comme le rapport de l'amplitude du mouvement des paupières sur la vitesse maximale de fermeture de celles-ci. Il y a corrélation entre l'augmentation de cet indicateur et le niveau de somnolence ainsi que la faculté à prédire des baisses de performance dans des tests de vigilance.
- Délai de réouverture des paupières : temps mesuré entre le début de la réouverture et l'atteinte de la vitesse maximale d'ouverture.
- Temps de fermeture et d'ouverture des paupières : durées de parcours des paupières, respectivement lors de leur fermeture et de leur réouverture, délimitées par les intersections de la vitesse de déplacement avec des seuils définis expérimentalement. Ces durées varient entre quelques millisecondes dans un état de plein éveil et plusieurs centaines de millisecondes lors d'épisodes de micro-sommeil.
- Période de clignement : intervalle de temps moyen séparant deux clignements consécutifs. La fréquence de clignement typique d'un adulte en plein éveil est généralement de 23 clignements par minute, et augmente avec la somnolence.
- Mouvements de la tête : variations de l'angle de la tête par rapport à sa position verticale normale, calculées à partir des accéléromètres et gyroscopes. Dans le cas de la conduite de véhicule, ces variations permettent de caractériser l'aptitude de l'individu à rester concentré sur la route devant lui, sans micro-chutes de tête caractéristiques d'un état de somnolence avancé et sans déport de l'attention prolongé vers des objets situés à l'intérieur de l'habitacle.

Les clignements ou battements de paupière (involontaires) sont des paramètres importants pour la détection de l'endormissement. Les clignements (battements) rapides et constants, le ratio pourcentage de fermeture / PERCLOS, et autres paramètres de la liste ci-dessus, sont des indicateurs fiables de la détection de l'endormissent.

Par ailleurs, le dispositif de l'invention permet de mesurer et déterminer la position des lunettes sur la tête de l'individu, et l'absence de port ou le port de ces dernières, comme montré sur l'organigramme de la figure 17, et illustré par les courbes de variation temporelle du signal filtré montré par les figures 17A et 17B.

Le signal issu du capteur infra rouge est d'abord filtré avec un filtre de moyenne glissante exponentielle (EMA en anglais), avec un coefficient faible, de l'ordre de 0.001.

Le signal résultant est ensuite injecté dans deux comparateurs en parallèle, qui compare le signal injecté à deux seuils K+ et K-, respectivement. Ces constantes sont choisies en fonction de la dynamique du signal et du coefficient du filtre ci-dessus.

Si les résultats de la comparaison sont « vrais », alors le multiplicateur correspondant est activé et la différence entre le signal brut et le signal filtré alimente un additionneur, relié à un dérivateur, lui-même relié à un déclencher d'événement.

Si aucun des comparateurs ne délivre un signal « vrai », alors le signal de sortie des multiplicateurs est égal à zéro.

Un seul comparateur peut délivrer un signal « vrai » à un instant donné.

Le signal alimentant le dérivateur est soit le signal filtré, soit la somme du signal filtré et la différence entre le signal brut et le signal filtré.

La détection des pics sur les graphes des figures 10A et 10B permet de détecter le port ou l'absence de port des lunettes.

Sur la figure 17A, on montre les courbes du signal respectivement filtré et brut. Les valeurs faibles du signal révèlent les périodes où les lunettes sont ôtées.

La figure 17B illustre la variation temporelle du signal à la sortie du dérivateur, qui montre clairement les périodes respectives de port (valeurs élevées) et d'absence (valeurs faibles ou nulles) de lunettes.

L'organigramme de la figure 13 montre les différentes parties de l'algorithme global, qui fait intervenir différentes sous-parties fonctionnelles, décrites ci-dessus, produisant des vecteurs de mesures référencés M(t), dont les composantes sont une série de scalaires mi(t), « i » étant la référence de la sous-partie correspondante, et « t » étant le temps.

Ces vecteurs mi(t) permettent de déterminer les différentes phases successives de l'endormissent d'un individu.

Le bloc fonctionnel D réalise la détection de l'endormissement sous la forme d'un vecteur d'état V(t), dont les composantes sont une série de scalaires vi(t) fonction des mesures à l'instant « t ». Les scalaires vi(t) sont calculés à partir des mesures et calculs réalisés par les sous-parties de l'algorithme.

Chaque composant vi(t) du vecteur d'état V(t) est mis à jour périodiquement, comme montré ci-dessous.

La fonction d'état $f$(t) prend la forme d'une combinaison linéaire des vecteurs d'état et de mesure, pondérés par des coefficients qui sont avantageusement ajustés (opération de calibrage) au fur e r à mesure des mesures, pour s'adapter à chaque individu et/ou conditions de mesures, d'environnement.

Les meures sont réalisées par exemple 100 fois par seconde. Les opérations de calibrage sont quant à elles réalisées 1 fois par jour par exemple

On définit :

$$V(t) = f(t) = \mathrm{A}.V(t-1) + \mathrm{B}.M(t)$$

$$\text{avec } \mathrm{A} = \begin{bmatrix} \alpha_{11} & \cdots & \alpha_{1n} \\ \vdots & \ddots & \vdots \\ \alpha_{n1} & \cdots & \alpha_{nn} \end{bmatrix}, \mathrm{B} = \begin{bmatrix} \beta_{11} & \cdots & \beta_{1p} \\ \vdots & \ddots & \vdots \\ \beta_{n1} & \cdots & \beta_{np} \end{bmatrix}, \alpha_{ij}, \beta_{ij} \in \mathbb{R}$$

Par exemple :

Supposons que la somnolence puisse être caractérisée par une augmentation de la fréquence moyenne des clignements d'oeil et une augmentation de la valeur moyenne de l'indicateur PERCLOS.

On note p(t) le temps séparant les deux derniers clignements d'oeil, a(t) l'indicateur PERCLOS du dernier clignement détecté, et z(t) l'estimateur de la somnolence.

On peut alors définir :

$$M(t) = \begin{bmatrix} p(t) \\ a(t) \end{bmatrix}, \qquad V(t) = \begin{bmatrix} \bar{p}(t) \\ \bar{a}(t) \\ z(t) \end{bmatrix},$$

$$A = \begin{bmatrix} \alpha_1 & 0 & 0 \\ 0 & \alpha_2 & 0 \\ \gamma_1 & \gamma_2 & \psi_1 \end{bmatrix}, \qquad B = \begin{bmatrix} \beta_1 & 0 \\ 0 & \beta_2 \\ 0 & 0 \end{bmatrix}$$

$$\beta_1 = 1 - \alpha_1, \qquad \beta_2 = 1 - \alpha_2, \qquad \psi_1 = (1 - \gamma_1 - \gamma_2)$$

On a alors :

$$V(t) = A.V(t-1) + B.M(t) = \begin{bmatrix} \bar{p}(t) \\ \bar{a}(t) \\ z(t) \end{bmatrix} = \begin{cases} \alpha_1.\bar{p}(t-1) + \beta_1.p(t) \\ \alpha_2.\bar{a}(t-1) + \beta_2.a(t) \\ \gamma_1.\bar{p}(t-1) + \gamma_2.\bar{a}(t-1) + \psi_1.z(t-1) \end{cases}$$

On choisit par exemple les coefficients suivants :

$$\alpha_1 = \alpha_2 = 0.9$$

$$\gamma_1 = 0.2, \qquad \gamma_2 = 0.6$$

Ce qui produit un lissage adapté au temps de réponse et à la sensibilité recherchés, et donne plus de poids à l'indicateur PERCLOS qu'à la fréquence de clignement.

On peut alors définir un seuil de déclenchement d'alerte $\Omega_{\text{alerte}}$ propre à chaque individu.

La valeur de ce seuil est calculée pendant une phase de calibration où l'individu est en plein éveil, à partir de la moyenne de z(t) :

$$\Omega_{\text{alerte}} = 1{,}4\,\overline{z_{\text{éveil}}}$$

Il est à noter qu'au-delà du traitement du signal instantané, cette forme de la fonction d'état permet de réaliser des fonctions de retard et stockage afin d'utiliser des valeurs ultérieurement.

Par exemple pour stocker les trois valeurs les plus récentes de la mesure $m_1$ dans $v_{1..3}$, on définit :

$$A = \begin{bmatrix} 0 & 0 & 0 & & 0 \\ 1 & 0 & 0 & \cdots & 0 \\ 0 & 1 & 0 & & 0 \\ \vdots & & \ddots & & \vdots \\ & \alpha_{n1} & & \cdots & \alpha_{nn} \end{bmatrix} \quad et \quad B = \begin{bmatrix} 1 & 0 & 0 & & \\ 0 & 0 & 0 & \cdots & \beta_{1p} \\ 0 & 0 & 0 & & \\ \vdots & & & \ddots & \vdots \\ & \beta_{p1} & & \cdots & \beta_{pp} \end{bmatrix}$$

$$V(t) = \begin{cases} v_1(t) = m_1(t) \\ v_2(t) = v_1(t-1) = m_1(t-1) \\ v_3(t) = v_2(t-1) = m_1(t-2) \end{cases}$$

On a alors

**Revendications**

1. Dispositif (100, 200, 300, 400) de suivi comportemental et de mesures de paramètres physiques d'un individu (110, 210, 310, 410), comprenant des moyens de détection et de mesure de paramètres représentatifs des mouvements et positions des paupières et des yeux de l'individu portant sur la tête ledit dispositif, et comprenant :

   - un émetteur de lumière (7, 8, 10, 11), suivant une direction dirigée vers au moins un oeil, lorsque le dispositif est porté ;
   - un capteur de la lumière réfléchie (9, 12) par ledit oeil, délivrant un signal variable en fonction de la couverture de l'oeil par la paupière
   - le dispositif étant apte à être installé, monté, fixé sur un appareil apte à être porté sur la tête d'un individu à proximité des yeux

   **caractérisé en ce que** les moyens de traitement et de comparaison comprennent :

   - un filtre de moyenne glissante exponentielle du signal issu du capteur, dit signal brut ;
   - un additionneur apte à calculer la différence entre le signal filtré et le signal brut ;
   - deux comparateurs (K+, K-) aptes à comparer le signal filtré à des constantes ;
   - deux multiplicateurs aptes à multiplier le résultat de la différence entre le signal filtré et le signal brut par le résultat de chaque comparateur ;
   - un additionneur pour additionner les résultats des deux multiplicateurs ;
   - un dérivateur pour dériver le signal ainsi obtenu ;
   - des moyens de détection pour détecter à partir du signal dérivé le port effectif du dispositif sur la tête de l'individu.

2. Dispositif selon la revendication 1, **caractérisé en ce que** le dispositif comprend une monture et lunette (2) et au moins un verre monté sur ladite monture et couvrant l'un au moins des yeux de l'individu.

3. Dispositif selon la revendication 1, dans lequel les moyens de mesure de paramètres physiques de l'individu comprennent la mesure des données suivantes :

   - le rythme cardiaque,
   - la température corporelle,

   et la mesure de l'une au moins des données suivantes :

   - les déplacements linéaires de l'individu selon trois axes,
   - les mouvements de rotation de la tête de l'individu selon trois axes,
   - les mouvements des pupilles et les clignements des yeux,
   - l'oxymétrie,
   - la pression artérielle,
   - la glycémie.

4. Dispositif selon l'une des revendications 1 à 3, comprenant un ou plusieurs moyens de communication ou de mesure choisis parmi :

   - des moyens d'envoi des données physiques de l'individu et de leur analyse vers une plateforme de stockage,
   - des moyens de mesure des données relatives à l'environnement extérieur suivantes : la direction du Nord magnétique, la température extérieure, la pression extérieure, la qualité de l'air, l'indice UV, l'altitude, la position géographique.
   - des moyens d'alerte de l'individu ou d'un ou plusieurs autres individus,
   - des moyens de stockage et/ou d'administration de médicament comme de l'insuline.

5. Dispositif selon la revendication 4, dans lesquel les moyens de mesure de données physiques relatives aux déplacements linéaires selon trois axes et aux mouvements de rotation de la tête selon trois axes de l'individu comprennent un gyroscope trois axes (23, 122, 222) et un accéléromètre (15, 13, 113, 121, 221).

6. Dispositif selon l'une quelconque des revendications 1 à 5, **caractérisé en ce que** les moyens de restitution de l'analyse vers l'individu comprennent un ou plusieurs moteurs (130, 230, 330) générant la vibration du dispositif et/ou un signal vibratoire et/ou un son.

7. Dispositif selon l'une quelconque des revendications 2 à 6, dans lequel les moyens de restitution de l'analyse vers l'individu comprennent un système lumineux (131, 231, 331) générant un signal visible sur le verre du dispositif dans le champ de vision de l'individu ou directement sur la rétine de l'individu.

8. Dispositif selon l'une quelconque des revendications 2 à 7, dans lequel moyens de restitution de l'analyse vers l'individu comprennent un projecteur générant des images sur le verre du dispositif dans le champ de vision de l'individu ou directement sur la rétine de l'individu.

9. Dispositif selon l'une quelconque des revendications 1 à 8, dans lequel l'analyse des données aboutit à la détection ou à la prédiction de l'endormissement de l'individu au volant d'un véhicule, l'individu portant le dispositif sur la tête.

10. Dispositif selon l'une des revendications 1 à 9, dans lequel pour chaque oeil, sont prévus un capteur (9,12) et deux moyens émetteurs (7, 8, 10, 11) ces deux derniers étant distants de part et d'autre d'une direction sensiblement horizontale, correspondant à l'axe optique de l'individu lorsque le dispositif est porté.

11. Dispositif selon la revendication 10, dans lequel les émetteurs de lumière (7, 8, 10, 11) et le capteur optique (9,12) sont du type infrarouge.

12. Dispositif selon la revendication 9, dans lequel les moyens de traitement et de comparaison comportent des moyens de détection de paramètres représentatifs de l'endormissement comportant :

   - un filtre du type " Butterworth "
   - un module de calcul de la dérivée du signal, dont les deux sorties sont respectivement reliées à :

      1. un élément de détection de pic de signal, relié à un moyen de stockage de données, lui-même relié à un comparateur binaire, débouchant sur deux éléments respectivement de comptage de la durée d'ouverture / de fermeture de la paupière ;
      2. un élément de calcul de la variance de la dérivée, relié à un premier comparateur avec un seuil, débouchant sur un compteur, lui-même relié à un élément apte à rechercher le dernier pic de signal (dans le moyen de stockage).

13. Dispositif selon la revendication 12, dans lequel lesdits moyens de traitement et comparaison comportent des moyens de détection de clignement de paupière involontaire, incluant successivement :

   - un filtre du type " Butterworth "
   - un élément de calibration, relié à un moyen de stockage des paramètres de l'algorithme de traitement
   - un moyen de détection de pic de signal
   - un élément comparatif de la valeur du pic à une valeur étalon / seuil
   - un élément de confirmation de pic, représentatif d'un clignement de paupière
   - un organe de traitement des données relié à un moyen de stockage.

14. Dispositif selon la revendicaton12, dans lequel les moyens de traitement et comparaison comportent des moyens de détection de clin d'oeil (volontaire) incluant :

   - un filtre du type " Butterworth "
   - un élément de calibration (pour tenir compte des particularités de l'individu porteur)
   - un moyen de détection de pic de signal
   - un élément comparatif de la valeur du pic à une valeur étalon / seuil
   - un élément de confirmation de pic, représentatif d'un clin d'oeil (volontaire).

**Patentansprüche**

1. Vorrichtung (100, 200, 300, 400) zur Verhaltensüberwachung und zu Messungen physischer Parameter eines Individuums (110, 210, 310, 410), die Mittel zur Detektion und Messung von Parametern umfasst, die für Bewegungen und Positionen der Lider und Augen des Individuums, das die Vorrichtung am Kopf trägt, repräsentativ sind, und umfasst:

- einen Lichtemitter (7, 8, 10, 11), der einer Richtung folgt, die auf mindestens ein Auge gerichtet ist, wenn die Vorrichtung getragen wird;
- einen Sensor (9, 12) für das vom Auge reflektierte Licht, der ein Signal liefert, das abhängig von der Bedeckung des Auges durch das Lid variabel ist,
- wobei die Vorrichtung dazu geeignet ist, an einem Gerät, das zum Tragen am Kopf eines Individuums in der Nähe der Augen geeignet ist, installiert, montiert, befestigt zu werden,

**dadurch gekennzeichnet, dass** die Verarbeitungs- und Vergleichsmittel umfassen:

- ein exponentiell gleitendes Mittelwertfilter für das vom Sensor ausgegebene Signal, Rohsignal genannt;
- einen Addierer, der dazu geeignet ist, die Differenz zwischen dem gefilterten Signal und dem Rohsignal zu berechnen;
- zwei Komparatoren (K+, K-), die dazu geeignet sind, das gefilterte Signal mit Konstanten zu vergleichen;
- zwei Multiplizierer, die dazu geeignet sind, das Ergebnis der Differenz zwischen dem gefilterten Signal und dem Rohsignal mit dem Ergebnis jedes Komparators zu multiplizieren;
- einen Addierer zum Addieren der Ergebnisse der beiden Multiplizierer;
- ein Differenzierglied, um das so erhaltene Signal abzuleiten;
- Detektionsmittel zur Detektierung des effektiven Tragens der Vorrichtung am Kopf des Individuums auf Grundlage des abgeleiteten Signals.

2. Vorrichtung nach Anspruch 1, **dadurch gekennzeichnet, dass** die Vorrichtung ein Brillengestell (2) und mindestens ein Glas umfasst, das im Gestell montiert ist und mindestens eines der Augen des Individuums bedeckt.

3. Vorrichtung nach Anspruch 1, wobei die Mittel zur Messung physischer Parameter die Messung der folgenden Daten umfassen:

- Herzrhythmus,
- Körpertemperatur,

und die Messung mindestens eines der folgenden Daten:

- lineare Verlagerungen des Individuums in drei Achsen,
- Drehbewegungen des Kopfes des Individuums in drei Achsen,
- Bewegungen der Pupillen und Lidschläge der Augen,
- Oximetrie,
- Blutdruck,
- Blutzucker.

4. Vorrichtung nach einem der Ansprüche 1 bis 3, die ein oder mehrere Kommunikationsmittel oder Mittel umfasst, die ausgewählt sind aus:

- Mitteln zur Sendung der physischen Daten des Individuums und ihrer Analyse an eine Speicherplattform,
- Mittel zur Messung der folgenden Daten einer äußeren Umgebung: Richtung des magnetischen Nordpols, Außentemperatur, Außendruck, Luftqualität, UV-Index, Höhe, geografische Position,
- Mittel zur Warnung des Individuums oder eines oder mehrerer Individuen,
- Mittel zur Lagerung und/oder Verabreichung eines Medikaments wie etwa Insulin.

5. Vorrichtung nach Anspruch 4, wobei die Mittel zur Messung physischer Daten der linearen Verlagerungen in drei Achsen und der Drehbewegungen des Kopfes in drei Achsen des Individuums ein dreiachsiges Gyroskop (23, 122, 222) und einen Beschleunigungsmesser (15, 13, 113, 121, 221) umfassen.

6. Vorrichtung nach einem der Ansprüche 1 bis 5, **dadurch gekennzeichnet, dass** die Mittel zur Wiedergabe der Analyse an das Individuum einen oder mehrere Motoren (130, 230, 330) umfassen, die die Vibration der Vorrichtung und/oder ein Vibrationssignal und/oder einen Ton erzeugen.

7. Vorrichtung nach einem der Ansprüche 2 bis 6, wobei die Mittel zur Wiedergabe der Analyse an das Individuum ein Leuchtsystem (131, 231, 331) umfassen, das ein Signal erzeugt, das auf dem Glas der Vorrichtung im Blickfeld des Individuums oder direkt auf der Netzhaut des Individuums sichtbar ist.

8. Vorrichtung nach einem der Ansprüche 2 bis 7, wobei die Mittel zur Wiedergabe der Analyse an das Individuum einen Projektor umfassen, der Bilder auf dem Glas der Vorrichtung im Blickfeld des Individuums oder direkt auf der Netzhaut des Individuums erzeugt.

9. Vorrichtung nach einem der Ansprüche 1 bis 8, wobei die Analyse der Daten zur Detektion oder Vorhersage des Einschlafens des Individuums am Steuer eines Fahrzeugs führt, wobei das Individuum die Vorrichtung am Kopf trägt.

10. Vorrichtung nach einem der Ansprüche 1 bis 9, wobei für jedes Auges ein Sensor (9, 12) und zwei Emittermittel (7, 8, 10, 11) vorgesehen sind, wobei die beiden Letzteren beidseitig von einer im Wesentlichen horizontalen Richtung entfernt sind, die der optischen Achse des Individuums entsprecht, wenn die Vorrichtung getragen wird.

11. Vorrichtung nach Anspruch 10, wobei die Lichtemitter (7, 8, 10, 11) und der optische Sensor (9, 12) vom Infrarottyp sind.

12. Vorrichtung nach Anspruch 9, wobei die Verarbeitungs- und Vergleichsmittel Mittel zur Detektion von Parametern umfassen, die für das Einschlafen repräsentativ sind, und umfassen:

- ein Filter vom "Butterworth"-Typ
- ein Modul zur Berechnung der Ableitung des Signals, dessen beide Ausgänge jeweils verbunden sind mit:

1. einem Element zur Signalspitzendetektion, das mit einem Datenspeichermittel verbunden ist, das wiederum mit einem binären Komparator verbunden ist, der in zwei jeweiligen Elementen zur Zählung der Dauer der Öffnung/Schließung des Lids mündet;
2. einem Element zur Berechnung der Varianz der Ableitung, das mit einem ersten Komparator mit einem Schwellenwert verbunden ist, der in einen Zähler mündet, der wiederum mit einem Element verbunden ist, das dazu geeignet ist, die letzte Signalspitze (im Speichermittel) zu suchen.

13. Vorrichtung nach Anspruch 12, wobei die Verarbeitungs- und Vergleichsmittel Mittel zur Detektion eines unwillkürlichen Lidschlags umfassen, die nacheinander aufweisen:

- ein Filter vom "Butterworth"-Typ
- ein Kalibrierelement, das mit einem Mittel zur Speicherung der Parameter des Verarbeitungsalgorithmus verbunden ist
- ein Mittel zur Signalspitzendetektion
- ein Element zum Vergleich des Spitzenwertes mit einem Richt-/Schwellenwert
- ein Element zur Bestätigung der Spitze, die für einen Lidschlag repräsentativ ist
- ein Organ zur Datenverarbeitung, das mit einem Speichermittel verbunden ist.

14. Vorrichtung nach Anspruch 12, wobei die Verarbeitungs- und Vergleichsmittel Mittel zur Detektion eines (willkürlichen) Lidschlags aufweisen:

- ein Filter vom "Butterworth"-Typ
- ein Kalibrierelement (zur Berücksichtigung der Besonderheiten des Trägerindividuums)
- ein Mittel zur Signalspitzendetektion
- ein Element zum Vergleich des Spitzenwertes mit einem Richt-/Schwellenwert
- ein Element zur Bestätigung der Spitze, die für einen (willkürlichen) Lidschlag repräsentativ ist.

**Claims**

1. Device (100, 200, 300, 400) for behaviourally monitoring and for measuring physical parameters of an individual (110, 210, 310, 410), comprising means for detecting and for measuring parameters which are representative of the movements and positions of the eyelids and of the eyes of the individual wearing said device on their head, and comprising:

- an emitter (7, 8, 10, 11) of light, in a direction directed towards at least one eye, when the device is worn;
- a sensor (9, 12) of the light reflected by said eye, delivering a signal which may vary as a function of the covering of the eye by the eyelid;
- the device being able to be installed on, mounted on, attached to a device which is able to be worn on the head of an individual in the vicinity of the eyes;

**characterized in that** the processing and comparison means comprise:

- an exponential moving average filter of the signal originating from the sensor, referred to as the raw signal;
- an adder which is able to compute the difference between the filtered signal and the raw signal;
- two comparators (K+, K-) which are able to compare the filtered signal with constants;
- two multipliers which are able to multiply the result of the difference between the filtered signal and the raw signal by the result from each comparator;
- an adder for adding the results from the two multipliers;
- a differentiator for differentiating the signal thus obtained;
- detection means for detecting, on the basis of the differentiated signal, whether the device is actually being worn on the head of the individual.

2. Device according to Claim 1, **characterized in that** the device comprises a frame and eyeglass (2) and at least one lens mounted on said frame and covering at least one of the eyes of the individual.

3. Device according to Claim 1, wherein the means for measuring physical parameters of the individual comprise measuring the following data:

- heart rate,
- body temperature,

and measuring at least one of the following data:

- the linear movements of the individual along three axes,
- the rotational movements of the head of the individual along three axes,
- the movements of the pupils and the blinking of the eyes,
- oximetry,
- blood pressure,
- glycaemia.

4. Device according to one of Claims 1 to 3, comprising one or more communication or measurement means chosen from among:

- means for sending the physical data of the individual and their analysis to a storage platform,
- means for measuring the following data relating to the external environment: the direction of magnetic north, the external temperature, the external pressure, the air quality, the UV index, the altitude, the geographical position,
- means for alerting the individual or one or more other individuals,
- means for storing and/or for administering a medicine such as insulin.

5. Device according to Claim 4, wherein the means for measuring physical data relating to the linear movements along three axes and to the rotational movements of the head along three axes of the individual comprise a three-axis gyroscope (23, 122, 222) and an accelerometer (15, 13, 113, 121, 221).

6. Device according to any one of Claims 1 to 5, **characterized in that** the means for rendering the analysis to the individual comprise one or more motors (130, 230, 330) generating the vibration of the device and/or a vibratory signal and/or a sound.

7. Device according to any one of Claims 2 to 6, wherein the means for rendering the analysis to the individual comprise a lighting system (131, 231, 331) generating a visible signal on the lens of the device in the field of vision of the individual or directly on the retina of the individual.

8.  Device according to any one of Claims 2 to 7, wherein means for rendering the analysis to the individual comprise a projector generating images on the lens of the device in the field of vision of the individual or directly on the retina of the individual.

9.  Device according to any one of Claims 1 to 8, wherein the analysis of the data results in the detection or in the prediction of the individual falling asleep at the steering wheel of a vehicle, the individual wearing the device on their head.

10. Device according to one of Claims 1 to 9, wherein, for each eye, a sensor (9, 12) and two emitting means (7, 8, 10, 11) are provided, the latter two being at a distance on either side of a substantially horizontal direction, corresponding to the optical axis of the individual when the device is worn.

11. Device according to Claim 10, wherein the emitters (7, 8, 10, 11) of light and the optical sensor (9, 12) are of the infrared type.

12. Device according to Claim 9, wherein the processing and comparison means comprise means for detecting parameters which are representative of falling asleep comprising:

    - a Butterworth filter
    - a module for computing the derivative of the signal, the two outputs of which are respectively connected to:

        1. an element for detecting a signal peak, connected to a data storage means, itself connected to a binary comparator, leading to two elements for counting the duration of the opening/closure of the eyelid, respectively;
        2. an element for computing the variance of the derivative, connected to a first comparator with a threshold, leading to a counter, itself connected to an element which is able to search for the last signal peak (in the storage means).

13. Device according to Claim 12, wherein said processing and comparison means comprise means for detecting involuntary blinking of an eyelid, successively including:

    - a Butterworth filter
    - a calibration element, connected to a means for storing the parameters of the processing algorithm
    - a means for detecting a signal peak
    - an element comparing the value of the peak with a benchmark/threshold value
    - an element for confirming a peak, which is representative of a blinking of an eyelid
    - a member for processing the data which is connected to a storage means.

14. Device according to Claim 12, wherein the processing and comparison means comprise means for detecting a (voluntary) wink including:

    - a Butterworth filter
    - a calibration element (for taking account of the peculiarities of the individual who is the wearer)
    - a means for detecting a signal peak
    - an element comparing the value of the peak with a benchmark/threshold value
    - an element for confirming a peak, which is representative of a (voluntary) wink.

FIG. 1

FIG. 2

FIG. 3

FIG. 4

EP 3 463 051 B1

FIG. 5

FIG. 7

FIG. 6

Fig. 11

Fig. 9

Fig. 8

Fig. 8

Fig. 10

Fig. 14A

Fig. 14B

Fig. 14

Capteur
Infrarouge
(données brutes)

Filtre passe-bas
Butterworth

X(t)

Calibration
des paramètres

Calibration
correcte ?

Détection
de pic

pic(t) > A

Calcul de la
dérivée

D(t)

Clignement d'oeil
détecté

Calcul
Des paramètres

Stockage des
paramètres de
l'algorithme :
• Δ : seuil de
  détection de
  clignement
  d'oeil

Stockage des paramètres relatifs au clignement d'oeil :
• Durée du clignement d'oeil
• PERCLOS 80
• Vitesse maximale de fermeture (PCV)
• Amplitude du pic (A)
• Rapport vitesse/amplitude (AVR)
• Vitesse maximale d'ouverture (PAV)
• Délai de réouverture de la paupière
• Temps de fermeture de l'oeil
• Temps d'ouverture de l'oeil
• Fréquence de clignements de l'oeil

Fig 15

Fig 15A.

Capteur infrarouge
(données brutes)

Filtre passe-bas
Butterworth

Calibration des
paramètres

Calibration
effectuée ?

Détection des
pics

pic(t) > A

Clin d'oeil
détecté

Stockage
des
paramètres
des
algorithmes

• Δ : seuil
  de
  détection
  du clin
  d'oeil

Fig 16

30

Fig. 17A

Fig. 17B

Fig 18

Fig 19

**EP 3 463 051 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2009105605 A
- EP 2061026 A
- US 2001028309 A
- US 2010134761 A